# EUROPEAN PATENT SPECIFICATION

(11) **EP 1 473 565 B1**
(45) Date of publication and mention of the grant of the patent: **25.08.2010**
(21) Application number: 04004322.6
(22) Date of filing: 16.07.1998
(51) Int. Cl.: G01N 33/543

(54) **A test device and method for detecting the presence of a residue analyte in a sample**
Testvorrichtung und Verfahren zum Nachweis von Restanalyten in Proben
Dispositif et procédé d'essai servant à détecter la présence d'un analyte ou d'un résidu dans un échantillon

(30) Priority: 16.07.1997 US 52644 P; 31.12.1997 US 1775; 11.06.1998 US 88937 P
(43) Date of publication of application: 03.11.2004
(62) Divisional of application: 98935709.0
(73) Proprietor: Charm Sciences Inc., Lawrence, MA 01843-1032 (US)
(72) Inventor: Markovsky, Robert J., Brentwood, NH 03833 (US); Francisco, Cheryl B., Fairhaven, MA 02179 (US); Charm, Stanley E., Boston, MA 02110 (US); Donahue, Paul R., Southboro, MA 01772 (US); Agi-Glickman, Yael, Brookline, MA 02146 (US); Saul, Steven J., Arlington, MA 02174 (US); Scheemaker, Joan L., Chelmsford, MA 01824 (US); Skiffington, Richard T., North Reading, MA 01869 (US); Trivedi, Shifali B., Quincey, MA 02170 (US); Zomer, Eliezer, Newton, MA 02158 (US)
(74) Representative: Price, Nigel John King

(56) References cited:
- EP-A- 0 299 428
- WO-A-93/17338

## Description

Lateral-flow or immunochromatographic test kits and methods for the detection of the presence or concentration of chemical residues or analytes or classes thereof from liquid samples have been developed. An example of one such test kit includes a pregnancy test kit.

Particularly in the food safety area it has long been recognized that residue detection should be accurate, inexpensive and easily conducted. Consumers and governments are becoming increasingly aware of the necessity for testing foods for the presence of undesirable residues naturally occurring or otherwise.

Since a large portion of the consumers are children, food safety has long been critical in the dairy industry. Antibiotic residues used on a dairy farm occasionally appear in the milk supply. The hazards associated with these undesirable residues, include allergic reactions, assisting the propagation of new and sometimes drug resistant microorganisms and other long term health risks.

Government agencies have established in some cases legal limits for particular residues in foods, for example antibiotic residues in milk. Residues above the "legal" limit are considered unsafe for human consumption. Residue levels below the legal limit are considered "safe". It is important, therefore, that detection methods, in addition to being inexpensive and easily conducted, do not give positive results when residues are below legal limits so that otherwise acceptable milk, or other foods, are not discarded or otherwise treated as containing residues above legal limits.

WO 93/17338 describes an assay for determining the presence of a threshold concentration of a single analyte in a sample which comprises first reacting the sample with an amount of anti-analyte selected to reduce the free analyte to a marginally detectable concentration then contacting the sample with anti-analyte immobilized on a test region on a solid phase.

### SUMMARY OF THE INVENTION

The invention relates to a method employing a lateral-flow test strip for the detection of the analyte or residue in a sample.

In its broadest form the invention is defined in independent claim 1:
A method employing a lateral flow test strip for determining whether one or more members of an analyte family comprising beta-lactam antibiotics is present in a test sample, which method comprises:
   a) allowing said sample to contact a microbial beta-lactam receptor, wherein said receptor is characterized by an ability to bind to members of said analyte family, so as to allow said receptor to bind one or more members of said analyte family in said sample;
   b) allowing said sample to contact an additional analyte binder, said additional analyte binder having binding specificity for at least one member of said analyte family to which said receptor binds, so that there is competition between the receptor and the additional analyte binder for binding to said at least one analyte, said competition causing a decrease in test sensitivity, relative to said at least one member, to a selected threshold level; and
   c) detecting said one or more members of said analyte family.

Often a liquid, such as milk, has one or more contaminants or analytes that are in trace amounts that need to be assayed. In order to detect the analyte, the present invention employs a labeled receptor that reacts with the analyte to form an analyte-receptor complex. The labeled receptor is positioned within or proximate to a membrane and when exposed to the liquid, lateral capillary flow occurs thereon. In the flow, the liquid carries the analyte-receptor complex, and any unbound labeled receptor with it. Positioned on the membrane in the flow path is a test zone. The test zone has a representative analyte conjugate attached to the membrane, to bind unbound receptor to form a first analyte conjugate receptor complex that, as a result of the label, has a signal visible to the eye or readable with an instrument.

Capillary flow of the liquid continues on the membrane to a control zone. The control zone includes a binder attached to the membrane that binds with the labeled receptor. Upon binding, the control zone changes to a signal that can be visible to the eye or readable with an instrument or visible under special light conditions, such as ultraviolet. If the signal in the test zone is more intense than the signal in the control zone, the test indicates that the analyte is not present in a sufficient amount (a negative test). If the test zone signal is less intense than the control zone signal, the test indicates that the analyte is present in an amount in excess of allowable levels (a positive test).

The receptor may bind a family of analytes (one or a plurality of analyte) which have similar structural binding sites. Members of an analyte family can have different detection level requirements and, therefore, additional analyte binders can be employed, for example, monoclonal or polyclonal antibodies, that bind a portion of the analyte in competition with the receptor, in the sample, thereby decreasing test sensitivity. The antibodies are mixed with the labeled receptor in an amount to adjust the sensitivity for a specific analyte or group of analytes. The sensitivity of the test is adjusted so that a positive test result is not given unless a certain threshold of analyte is present in the sample.

The test device includes a support strip and a sample-absorbing matrix attached to the support strip. The sample-absorbing matrix is a material for absorbing an amount of the sample for example a sponge. The test device also includes a mobile-phase support for holding a mobile-phase composition. The mobile-phase support is attached to the support strip and is in contact with the sample-absorbing matrix. A mobile-phase composition is disposed within or on the mobile-phase support and has a labeled receptor for binding with the analyte. The mobile-phase composition can be carried in the sample and flow together with the sample. A stationary-phase membrane is attached to the support strip and has a first membrane end in contact with the mobile-phase composition and a second membrane end in contact with the disposal zone. The membrane allows lateral capillary flow of the sample from the first membrane end to the second membrane end. A test zone is on the stationary-phase membrane between the first membrane end and second membrane end and has an analyte conjugate for binding with unbound labeled receptor. A control zone is on the stationary-phase membrane between the test zone and second membrane end and has a binder, for example an antibody to the particular receptor, for binding with analyte-bound receptor and excess unbound receptor.

The present disclosure also includes an analyte test device for detecting, in a general horizontal position, an analyte in a liquid sample by capillary lateral flow in a chromatographic test strip. The device includes an elongated housing defining an elongated strip cavity having an open application aperture at one end and having another end. The cavity is adapted to receive and hold a test strip therein. The housing has a transparent top cover section to allow the observation of tests results on the test strip. The housing is characterized by an enlarged application cavity extending outwardly from the top cover and having or adapted to have an open end at the application end. The test device includes a test strip positioned in the strip cavity.

The test strip includes a support strip with a plurality of sequential contacting, liquid-sample, permeable zones extending from the first end to the second end. The zones allow the lateral capillary flow of the liquid sample from the first end to second end. The zones include a sample-absorbing and filtering zone composed of an expandable, porous, compressed-material layer which moves, on contact with the liquid sample, between a nonexpanded state to an expanded state on absorption of a preselected amount of the liquid sample, and a mobile phase support having a mobile-phase composition layer thereon or therein with a labeled receptor for binding the analyte in the liquid sample thereon, typically a visible area containing colored beads and a membrane generally of nitrocellulose which includes a reaction zone having at least one stationary analyte conjugate reference or test line, or generally a test and a separate control line thereon and optionally a disposal zone of liquid-sample absorbent material to absorb less liquid sample and to aid in capillary flow to the second end.

The sample-absorbing zone with the compressed material layer is positioned adjacent the application cavity. The compressed-material layer and the application cavity are designed to allow the compressed-material layer to absorb a selected amount of liquid sample for testing and in an amount sufficient to carry out the test and to expand from a dry, nonexpanded form to a wet, expanded state. The material layer in a wet, expanded state fills substantially the application cavity and causes sufficient pressure on the housing walls of the expansion cavity to drive capillary flow of the liquid sample in the application cavity to a selected volume, when the open application end of the test device is inserted into a liquid to obtain the liquid sample or when a known amount of sample is pipetted into the application cavity.

In one embodiment, a housing is employed, such as a one-piece, integral, injection-molded, all-transparent, plastic material, with the plastic material selected or designed to be subject to incubator temperatures of 30°C or more for incubation times, for example, 2 to 10-15 minutes, depending on the particular test although not all tests will require incubation at temperatures other than room temperature.

In one embodiment, the housing includes a generally toothbrush shape, with an enlarged, generally triangular, toothbrush-type head at the open application end of the housing, with a dry, inert, porous, expanded, liquid-permeable, absorbing material in a generally triangular layer as an absorbing zone in the test strip, for example, of cellulose or nitrocellulose, positioned beneath the open bottom of the application cavity or chamber. The absorbing layer on contact, such as immersion of the application end of the housing of the test device in a liquid, absorbs a preselected amount of the liquid sample necessary for the test. The absorbing-layer material expands for example, in one to thirty seconds, to fill or substantially fill the expansion cavity and contact the surrounding walls of the expansion-cavity housing, to cause sufficient pressure within the expansion cavity and the expanded state of the material to drive capillary flow laterally in the underlying test strip laterally toward the end of the elongated housing where the test strip is positioned. The expansion cavity and underlying absorbing-layer material which generally mimics two dimensions of the expansion cavity, permit absorbing and filtering of the selected amount of liquid sample for the test strip. The expansion cavity and absorbing-layer material aid in driving the lateral flow of the liquid sample in the test strip in the housing toward the disposal zone at the end of the strip to receive the liquid sample where employed. If the absorbing layer does not expand sufficiently to fill or substantially fill the expansion cavity, the lateral or capillary flow rates and times can be unsatisfactory. The flow rate can be too slow and the time period can be too long. If the absorbing layer is used in excess, then excess pressure occurs in the expansion cavity, and the expanded absorbing layer tends to retard the desired lateral flow of the liquid sample.

The housing with the toothbrush-shaped design can include a separate injection-molded housing with an optional end cover, to protect the exposed application end before sampling and after sampling, and in the incubation chamber, to prevent cross-contamination from other sources. The test device with the molded housing allows the user to handle the handle end of the housing and to obtain a liquid sample merely by dipping the open application cavity into a liquid.

The housing can include a toothbrush-shaped design, wherein the expansion cavity is formed in a plastic, usually transparent, blister-type package which is sealed against a flat support, such as a paper strip or another plastic strip, and which encompasses within the blister package the selected test strip. The blister package includes a removable seal strip at the one application end of the enclosed test strip, for peeling or removal prior to use and for the introduction of a selected volume of the liquid to the application-absorbing zone of the test strip while in the blister package, e.g. by pipetting. The blister package with the liquid sample and test strip can be incubated in the incubator and the test results observed visually or read by an instrument.

In another embodiment, it has been discovered to be desirable to provide one or more apertures in the housing which defines the expansion cavity, to permit the time-controlled and more rapid absorbing of the liquid sample into the absorbing material for more efficient absorption and to reduce absorption time of the liquid sample. In particular, one or more apertures should be placed on the top cover or surface of the expansion-cavity housing, particularly of the molded housing, rather than on the sides, so that entrapped air after immersion is discharged from the expansion cavity, as the absorbing layer expands into the wet, absorbing, expanded state. While a flat, rectangular strip of absorbing material is shown with a generally rectangular expansion cavity which mimics and provides for the expanded, rectangular stip of the absorbing zone, it is recognized that the size, material, dimensions and shape of the absorbing material and the shape or form of the expansion cavity may vary. Typically, the open bottom of the expansion cavity is directly above the absorbing layer and usually of about the same width and length dimensions, to permit expansion without restriction of the absorption layer into the expansion cavity.

While a fully transparent top cover is desirable to enclose the test strip and observe or read the test results on the test strip, it is recognized that the top cover can be open or have an aperture to view the test results, or only a section of the top cover be transparent to view the test results, or where applicable the housing may be modified, so that the test results can be determined by optical or electronic instrument means.

The test device can be packaged for use in a blister-type package or employ a fixed or slidable protective cap at the application end, to protect the test device from contamination prior to use and to protect the test device after contact with the liquid sample and in the incubator (where required in the test), to protect against cross-contamination. The protective cap can be removable and enclose totally the application end of the housing, or merely be slidably extended outwardly from the application end between a retracted use position and extended, protective, closed position.

The test device employs a test strip selected to detect the presence or concentration of selected analytes or residues, either a single residue or classes thereof, and visually by reference of a reaction test zone or reference line in the test strip which may be observed or measured. Usually, a control zone or line is spaced apart slightly downstream from the reference zone or lines for control purposes. The housing of the test device is applicable to a wide variety of present employed or described test strips which are based on lateral flow or capillary flow, regardless of the nature of the particular analyte-residue test, provided only that the application or liquid contact portion of the test strip requires or uses a filtering absorbing material which moves by liquid-sample contact between a nonexpanded and an expanded state at or toward the one application end of the test device. Typically, the test strip has a support and includes on one surface a plurality of contacting, liquid-permeable, sequential zones or sections with a stationary zone, a mobile zone and, optionally, a disposal zone. The test device is particularly useful in connection with the liquid sample comprising a fluid, for example, urine, blood, milk or corn extract and in the detection of antibiotics, like beta-lactams, toxins, viruses, bacteria, pesticides and the like. However, the test device can employ one or more test strips directed to a variety of tests.

Where applicable, the test device is employed in combination with an incubator, such as a portable, electrically heated incubator with an incubation chamber which can be dimensioned to receive the test-device housing snugly therein for heating for a selected incubator time, for example, at a temperature in the range of between about 45 and 75°C, preferably between 55 to 65°C, and for a period of 1 to 10-15 minutes. The test device and incubator also include a timer, so that the incubation period can be timed by a user.

In operation, the test device with a protective covering or cap has the cover or cap removed and the application end contacted with a liquid to be tested, such as by immersion, for about one to ten seconds and then removed, or a liquid sample pipetted into the application end. The absorbing material is allowed to expand within the expansion cavity, for example one to fifteen seconds, then the test device is placed in an incubator for a time period, then removed and the test results observed or measured. If the sample is pipetted the device is placed in the incubator and the sample is pipetted into the sample cavity.

The present disclosure includes many advantages such as combining high purity broad spectrum receptors or antibodies with high specific activity per surface area combined with counteracting residue specific antibodies (e.g. monoclonal) to achieve residue detection on the order of parts per billion (ppb) (10⁻⁹) or parts per trillion (ppt) (10⁻¹²) levels at or close to regulatory requirements. Targeting the active moiety of the chemical residue allows detection of a broad spectrum of active pharmaceuticals (e.g. veterinarian drugs, agricultural chemicals (e.g. pesticides), or microbial toxins and their active metabolites.) Further, additional antibodies can be added to adjust the threshold sensitivity of the test.

Other advantages include that all components can be incorporated in the device and reagent preparation is not necessary. The device is a one-step assay that does not require timing (results are stable from about four minutes to few hours). The device which has a built-in negative control eliminates the need for external control standards.

### BRIEF DESCRIPTION OF THE DRAWINGS

Figure 1 is a perspective, exploded view of a molded-housing test device.
Figures 2, 3 and 4 are schematic, illustrative view of the use of the test device of Figure 1.
Figure 5 is a perspective view of an incubator and the test device with a liquid sample.
Figure 6 is an enlarged, front-plan view of the test strip of Figures 1-5, with enlarged, front, sectional views of positive and negative test results.
Figure 7 is a perspective, exploded view of a blister-pack test device.
Figures 8, 9 and 10 are schematic, illustrative, side views of the use of the test device of Figure 7.
Figures 11 and 12 are perspective views of an incubator and the test device with a liquid sample.
Figure 13 is an enlarged, front-plan view of the test strip of Figures 7-12, with enlarged, front, sectional views of positive and negative test results.
Figure 14 is a perspective, exploded view of a blister-pack test device with protruding backing and narrowing of blister to produce pinch points.
Figures 15, 16 and 17 are schematic, illustrative, side views of the use of the test device of Figure 14.
Figure 18 is an enlarged view of the strip movement restriction zone 114.

### DETAILED DESCRIPTION OF THE INVENTION

The foregoing and other objects, features and advantages of the invention will be apparent from the following more particular description of preferred embodiments of the invention, as illustrated in the accompanying drawings in which like reference characters refer to the same parts throughout the different views. The drawings are not necessarily to scale, emphasis instead being placed upon illustrating the principles of the invention. All percentages and parts are by weight unless otherwise indicated.

A test device and method for detecting the presence of a residue analyte in a sample are described. The test and device use direct color, fluorescence or infrared recognition-based broad spectrum assays to rapidly detect low part per billion (ppb) presence of a chemical or a family of chemical residues sharing common recognition sites. The kits are designed for testing antibiotics, toxins and pesticides in food or environmental samples in the field, or in the lab. The assays are non-competitive using saturation chemistry.

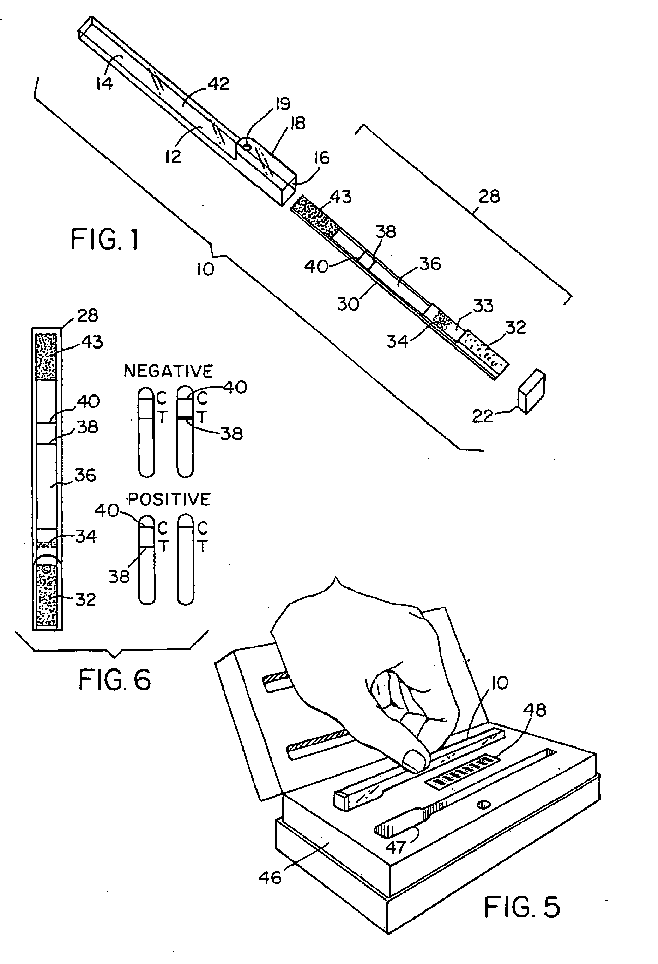

In the drawings, Figures 1-6 show analyte test device 10 which includes elongated, molded housing 12. Housing 12 can be formed of a one-piece, injection-molded, transparent styrene polymer. Housing 12 defines elongated housing cavity 14 with open end 16, and having enlarged, rectangular application expansion cavity 18 at open end 16 of housing 12. Housing 12 includes an elongated bottom cavity formed during the injection-molding process. The housing includes an optional removable, friction-fitted or snap-on protective cap 22 adapted to fit over open end 16 of housing 12 and liquid expansion apertures 19 in the top cover of the application housing cavity to increase the efficiency of expansion of a sample-absorbing matrix, such as sponge 32, within the test time.

Housing cavity 14 includes therein on the bottom surface a lateral-flow test strip 28 adapted to detect the presence of an analyte in a liquid sample, such as milk. Test strip 28 includes support strip 30 with sponge 32 attached at one end. Sponge 32 can include a plurality of sequential layers comprising a rectangular pad of dry, compressed, cellulosic material as a liquid-sample absorbent secured to the face surface of the support strip 30. Sponge 32 is selected to expand in contact with the liquid, such as milk, to fill the expansion cavity 18 which sponge 32 mimics in two dimensions. For example, with milk, sponge 32 is about 3-4 mm by 12-14 mm, while cavity 18 is about 5-6 mm by 15-16 mm by 4-6 mm in height. Expansion cavity 18 can be dimensioned about 60% to 30% less than the full expansion of the sponge material.

Support strip 30 includes treated, mobile-phase support 33 with mobile-phase composition 34, stationary-phase membrane 36 which includes test zone 38 and control zone 40 for the analyte to be detected, and disposal zone 43 at second end of support strip 30 to capture excess liquid sample. Housing 12 includes transparent top cover 42 for visual observation of test zone (reference zone) 38 and control zone 40. Test strip 28 is placed and positioned loosely in the elongated cavity 14, with sponge 32 positioned beneath the expansion cavity 18, and sponge 32 extending generally to about or slightly beyond the plane of the open application end, and the end covered prior to use by protective cap 22.

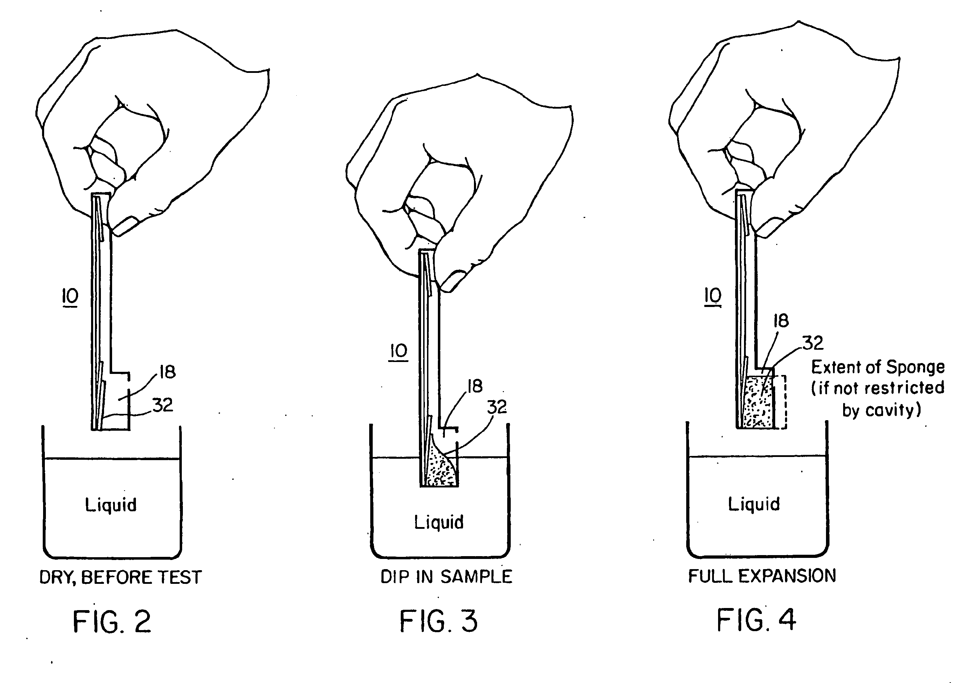

In operation, protective cap 22 is removed prior to use and the open application end of housing 12 inserted briefly (about one to ten seconds) in the liquid, such as milk, to be tested employing elongated housing 12 as a handle. See Figure 2. Test device 10 is removed and sponge 32 is allowed to expand to fill expansion cavity 18 and to start the lateral flow of the milk sample through test strip 28 (2 to 6 minutes). See Figures 3 and 4. Preferably, protective cap 22 is inserted to protected against cross-contamination, and test device 10 then placed in a horizontal position, with the application cavity 18 extending downwardly in an electric-heated incubator 46 with incubator cavity 47 shaped to receive the test device, and incubation carried out, for example, for three to ten minutes. The incubation temperature is observed through the temperature-indicator scale 48. See Figure 5. Incubated test device 10 is then removed and reversed, and the front view of the test device with test zone 38 and control zone 40 observed. See Figure 6. The line readings for positive and negative controls are illustrated in Figure 6 adjacent the front view of test device 10. In sponge 32, expansion is controlled by the expansion cavity 18 volume and size, resulting in sponge 32 completely filling expansion cavity 18 with a preselected volume of liquid, for example 0.1 to 1.0 ml, so the amount of liquid sample taken in for the test is controlled to the correct amount. The dimensions of expansion cavity 18 prevent the sponge pad 32 to fully expand, so that pressure is maintained in the expanded sponge, as shown in Figure 4, to aid in forcing capillary-lateral flow of the liquid sample through the test strip 28 in the housing 12.

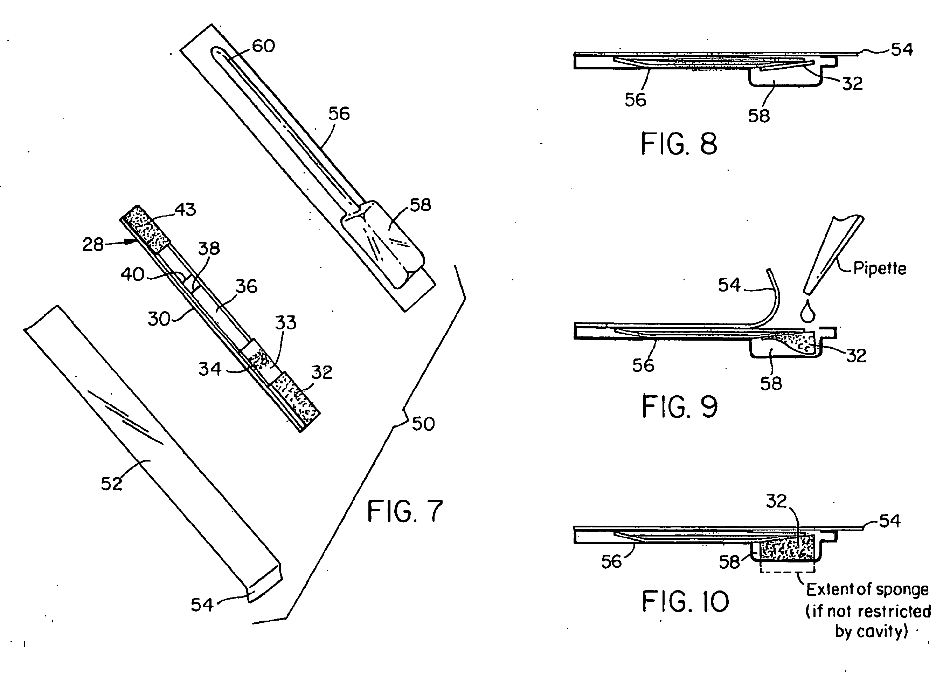

The drawings in Figures 7-13 illustrate another embodiment of test device 50 in a transparent blister package which includes transparent-tape plastic seal strip 52 with peel tag 54 at one end, and transparent blister package 56 adhesively secured to strip 52, to enclose test strip 28 therein. Blister package 56 includes an elongated cavity to hold strip 28 and an expansion cavity-housing 58 at the one end to form a generally toothbrush-shaped cavity within plastic blister package 56 and strip 52. Selected test strip 28 is sealed and enclosed within blister package 56.

Figure 8 shows a side sectional view of the blister-package test device 50 prior to use. Figure 9 shows blister-package test device 50 with one end peeled back by peel tab 54, to expose expansion housing cavity 58 and sponge 32 of test strip 28, so that a defined amount of a liquid sample can be added, for example, by pipet, as shown. In a preferred embodiment, cavity 18 is triangular-shaped similar to sponge 32 as shown in Figure 9.

Figure 10 illustrates test device 50 after addition of the liquid sample, and with peel tab 54 resealed and with sponge 32 fully expanded by the liquid sample within housing cavity 58 and ready to incubate.

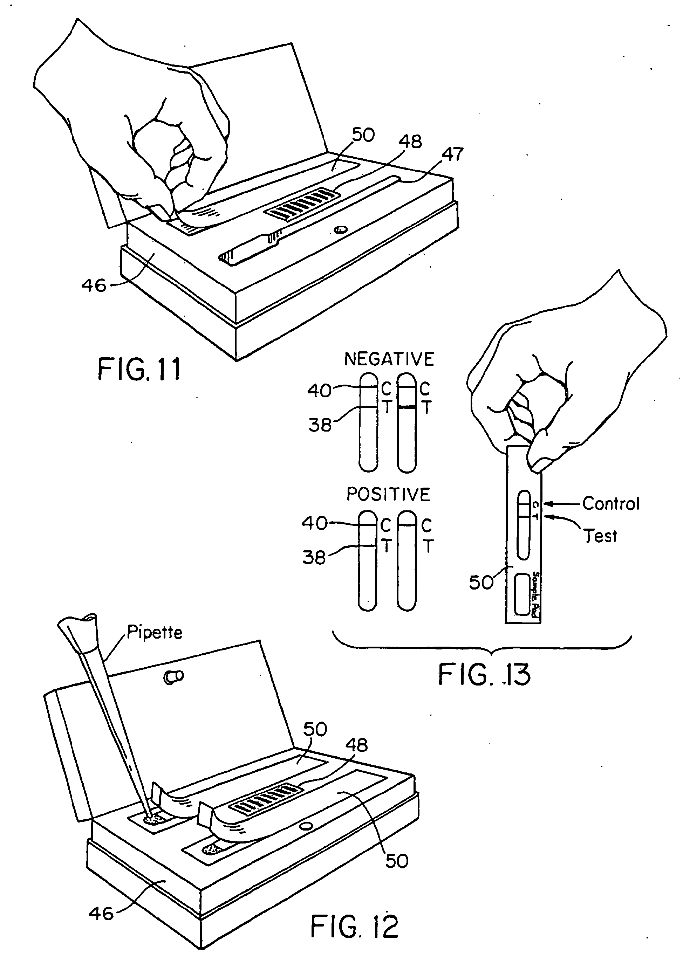

Figure 11 illustrates test device 50 upside down and placed in one of two cavities 47 in incubator 46. Figure 12 illustrates the technique of adding the liquid sample with a pipet, while peel tab 54 is pulled away from the end of test device 50 in incubator 46. Test device 50 is sealed and incubated. The test results of the completed test can then be read through a transparent top cover of blister package 56, as shown in Figure 13, to provide positive or negative test results.

Inhibition assay test strip 28 (Figure 7) selected for beta-lactams in milk is a quick test for beta-lactams in commingled raw and pasteurized milk. In operation, temperature gauge 48 in incubator 46 is checked to ensure an incubator temperature of about 55°C. For example, temperature indicator 48 may be colored, for example, green, for use. Test device 50 is placed in one cavity 47 of incubator 46 with the flat side facing up and peel tab 54 peeled back enough to expose sponge 32, for example one centimeter. The milk is mixed thoroughly before testing, and about 0.2-0.7 ml, preferably 0.3-0.5 ml, is added by pipet to exposed sponge 32. Adhesive tape tab 54 is resealed by hand pressure and incubator 46 cover is closed. Test device 50 is incubated, for example, at least 6 to 8 minutes and then removed from incubator 46 and held vertically and a comparison made within about one hour between test zone 38 and control zone 40. If no control zone 40 appears, the test is invalid. A negative test occurs when reference zone 38 is the same or darker than control zone 40. A positive test is indicated when test zone 38 is absent or clearly lighter than control zone 40.

In more detail, test device 10 capable of detecting analytes in biological fluids includes the following components:

Sponge 32, a compressed material, such as cellulose, is capable of absorbing a biological fluid and acting as a prefilter to remove coarse contaminants, such as hair, dirt, etc. Sponge 32 is sized to absorb a fixed amount of sample required to complete the assay. This compressed material, when expanded and contacting the inside wall of housing 12, causes sufficient pressure to drive capillary flow along the components sponge 32, mobile-phase support 33, stationary-phase membrane 36, and disposal zone 43 and in the time required (about 3 to 8 minutes) for a commercially marketable test. Sponge 32 overlaps mobile-phase support (conjugate pad) 33 by 1 to 10 mm such that, when an aqueous sample, such as milk, is added to sponge 32, the sample flows onto mobile-phase support 33.

The test device can use a biological receptor that is tagged with ample amounts of color, infrared, fluorescent or luminescent dyes. The liquified sample (e.g. milk, corn, feed, peanut extract, meat extract, serum, environmental sample, etc.) resuspends the tagged-receptor which is previously stabilized with readily soluble additives in a mobile-phase composition. The time controlled released tagged-receptor reacts with the analyte in the sample while moving into a reaction zone on stationary-phase membrane 36.

Residue specific monoclonal antibodies are also included in the mobile-phase composition to specifically bind excess residue with high sensitivity, thus, adjusting the sensitivity for those specific residues downward (to make the test less sensitive). As less of these residues are available to compete with the broad spectrum receptor, the sensitivity is adjusted closer to regulatory requirement. For example, initial sensitivity of beta-lactam receptor to cephapirin is 3-5 ppb. Including specific antibodies to cephapirin the sensitivity is adjusted to 15-20 ppb. Food and Drug Administration regulatory "safe" level in raw, commingled milk is 20 ppb.

It will be appreciated that specific antibodies complex with free antibiotics in a sample, making the complexed antibiotics unavailable for further measurement in any binding or receptor inhibition assay.

Housing 12 should be used to allow for addition of biological sample, either by dipping, pouring or pipetting. Housing 12 can be constructed of a flexible or hard material, such a polystyrene, polypropylene, or polyethylene.

Mobile-phase support 33 can be made of a glass membrane or a polymer, such as polyester or polyethylene, that acts as a secondary filter for removal of less coarse materials (somatic cells). The support is pretreated with a chemical solution, such as 0.01 to 0.2 M sodium citrate pH 6-8, capable of neutralizing interferences found in biological samples. The mobile-phase support overlaps stationary-phase membrane 36 (reaction strip) by about 1 to 4 mm.

The color or fluorescent receptor/antibody coated microspheres are suspended in a solution containing protein e.g. albumin bovine (BSA), glycerol, sugar or equivalent thereof e.g. sucrose (SUC) or trehalose (TRE), polyethylene glycol 8,000 MW (PEG), amino acid mixtures (AA) or detergents as stabilizers and wetting agents, and absorbed or sprayed in the membrane using a spraying instrument such as is available from Biodot. Furthermore, the residue specific antibodies are spray dried or immobilized in this matrix. Sample buffering is also optimized here using a buffer with a given pH, salt or any required cofactor needed to optimize the receptor/antibody binding kinetic.

A mobile-phase includes highly specific binding proteins, such as an enzyme, or monoclonal antibodies capable of binding to an analyte and titrated to a known concentration to make unavailable for further reaction/detection of a known amount of analyte. This unavailability for further reaction/detection allows for the adjustment of a detection level of one or more analytes to a specified level of concern. For example, in ceftiofur, a beta-lactam with a tolerance level of 50 ppb in milk, sensitivity can be changed from 5 ppb to between 40-50 ppb by the addition of a monoclonal antibody specific for ceftiofur. The specific monoclonal antibody competes with the labeled receptor to remove a specific analyte from binding to a receptor or antibody which is capable of binding to a family of related compounds.

Highly purified proteins, such as, beta-lactam receptor, prepared by affinity purification and/or a combination of hydrophobic/ion-exchange chromatography, are attached to a colored, fluorescent, or infrared probe which can be observed by optical/instrumental means or both. Attachment of proteins to a probe is called binding protein/probe complex.

Mobile-phase composition 34, such as gold beads, is made to a particle size between 10 and 60 nm. The beads can be sprayed onto a mobile-phase support 33, such as a pretreated high density polyethylene. The mobile-phase composition is sprayed, using a machine such as by Ivek, Biodot, or Camag, or absorbed onto sponge 32 in a solution containing 5 to 20% sugar, such as sucrose or trehalose, 5 to 20% protein, such as BSA or Primatone, 5 to 100 mm of a buffer solution (phosphate or Trizma base) with a final pH of between 6-8. The mobile-phase composition is sprayed on the upper portion of the mobile-phase support, such that sponge 32 does not overlap the mobile-phase composition sprayed portion of mobile-phase support by placing the topmost portion of sponge 32 1 to 7 mm before the sprayed portion of mobile-phase composition.

A stationary phase membrane can consist of nitrocellulose or nylon which has multiple reaction zones. The control zone, containing antibody to the receptor, is sprayed at the same time and the nitrocellulose is dried. Zone thickness is adjusted by added BSA to mobile-phase composition. The mobile-phase composition is sprayed on the upper portion of the mobile-phase support 33, such that mobile-phase composition 34 does not overlap sponge 32, but rather mobile-phase support 33 overlaps sponge 32 by placing the topmost portion of sponge 32 about 1 to 7 mm before the sprayed portion of mobile phase. The mobile-phase support is dried and tested in an assay system for sensitivity of all the drugs. Antibody concentrations in the mobile-phase composition solution are then adjusted as needed.

For the mobile phase to obtain the best color or fluorescence response, highly purified proteins (beta-lactam receptor example) can be prepared by (I) affinity purification and/or (ii) a combination of hydrophobic / ion exchange chromatography.

For color, infrared, or fluorescent probes, the receptor/antibody can be either a color, infrared, or fluorescent dye immobilized in, for example, a 0.02-10 micrometer microsphere or colloidal gold. These supports either absorb protein or exhibit functional groups such as NH2, COOH or CHO for covalent protein binding. The microsphere generally is coated with high concentration of highly specific binding proteins.

Typically, the stationary-phase membrane is formed of nitrocellulose, nylon, polyethylene or another suitable material. In the stationary phase, the analyte representative drug is attached with high specific ratio to a carrier e.g. a protein such as BSA, IgG or Protein A. Stationary-phase membrane 36 has multiple reaction zones present and includes test zone 38 sprayed in a line using a suitable spraying instrument. The purpose of the test zone is to capture unreacted binding protein/probe complex for viewing or measurement. Test zone 38 (Figure 6) consists of an analyte of detection; that is, ceforanide or a member of the analyte family, that is, beta-lactams, coupled to a carrier protein, that is BSA, IgG, KLH, suspended in a 5 to 100 mM buffer solution (such as phosphate or buffer base) at a pH range of 3-10, preferably 6-8. Total protein concentration of the antibody solution ranges from 0.2 to 100 mg/ml. The analyte-carrier, dissolved in a buffer solution e.g. 10 mM phosphate buffer, pH 6.9 containing sugar, such as trehelose or other additives, is sprayed as a line on the stationary-phase membrane. Tentacle immobilization of analyte conjugate to a multiple binding site carrier, such as Protein A or latex microspheres, increases stability and binding capacity. Subsequent heat treatment of the membrane further stabilizes the adhesion. On the test device, a second test zone can be added to the reaction zone to test for a second analyte. For example, the first test zone can have a first binder for amoxicillin, ampicillin, ceftiofur, cephaparin and penicillin G. The second test zone can have a second binder for cloxacillin. Alternatively, the first test zone can test for beta-lactams and the second test zone can test for sulfonamides. Additional test zones can be added to test for additional analytes.

The reaction zone also includes control zone 40, shown in Figure 13, sprayed in a line form using a suitable spraying instrument. A purpose of control zone 40 is to capture binding protein/probe complex that has not bound to test zone 38. Control zone 40 can consist of an antibody specific to the binding protein/probe suspended in 5 to 100 mM of a buffer solution (phosphate or Trizma) in a pH range of 3 to 10. Total protein concentration of antibody solution ranges generally from 0.2 to 100 mg/ml.

In one embodiment, the material for ceforanide-SM is added to the SMCC-BSA-NEM solution and the reaction continues with stirring overnight at 4°C. The ceforanide-BSA is dialyzed to remove free ceforanide. Control zone includes an antibody to the tagged receptor or broad spectrum antibody that is immobilized as a line parallel to the test zone. Thus, mobile-phase composition receptor/antibody captures in this line regardless of presence or lack or analyte in the sample. The control zone consists of an antibody made to the beta-lactam receptor. The receptor is purified by affinity chromatography.

A comparison of the control zone to the test zone yields the test result. Typically, if the control zone is darker than the test zone, analyte is present at detection level or greater (see Figure 6).

Disposal zone 43, shown in Figure 7, typically is made of pressed cellulose or other absorbent material to keep the sample flow consistent and to retain the reacted sample. The disposal zone generally overlaps the stationary-phase membrane 36 by about 1 to 5 mm.

The mobility of the sample (milk, blood serum or other fluids) is tested to optimize reaction times and uniformity. High pore size membranes (15 to 140 µm) are used to allow flow of viscous samples like milk or serum.

The disposal zone 43 typically includes an absorbent pad that is an absorbing membrane made of a cellulose, synthetic sponge or other material. This pad keeps the sample flowing and stops flow at saturation, thus giving the assay time control and reducing background noise.

In another specific embodiment, an aqueous biological sample is added to sponge 32 of the test device. Sponge 32 serves as a sample pad which expands as it absorbs the sample. Sponge pad 32 overlaps mobile-phase support 33, and the fluid flows onto the mobile-phase support 33 where the mobile-phase materials dissolve into the biological fluid. Analytes present in the sample begin binding with the specific binding protein(s) attached to the probe. At the same time, specific bound or unbound antibodies or binding proteins bind with specific analytes to adjust their sensitivity to the test. Mobile-phase support 33 overlaps stationary-phase membrane 36, and the biological fluid, along with mobile-phase composition 34 (colored beads), continue to react as materials flow up stationary-phase membrane 36. When the binding protein/probe complex reaches test zone 38, a portion of the binding protein/probe complex binds to the test zone. In a positive sample, analyte in the sample is bound to the binding protein/probe complex, reducing the amount of binding protein/probe complex capable of binding to the test zone 38. When the material reaches control zone 40, a portion of binding protein/probe complex binds control zone 40. Excess reagent is then absorbed into disposal pad 43.

In a negative sample, reagents are titrated so that test zone 38 has the same or preferably a greater amount of the probe binding to it than in control zone 40. Conversely, in a positive sample, control zone 40 has a greater amount of the probe binding to it than test zone 38.

In still another embodiment, a beta-lactam test is made to assay for beta-lactams in milk at a safe level. A partially purified beta-lactam receptor from BST (*Bacillus stearothermophilus*) is bound to a colloidal gold solution to make a beta-lactam binding protein/gold bead probe. This is sprayed on the mobile-phase support 33 along with monoclonal antibodies to ceftiofur, cephapirin, ampicillin and amoxicillin to reduce the sensitivity of these four antibiotics so that the test gives a desired dose response. On test zone 38 is sprayed a ceforanide-BSA conjugate, and to control zone 40 is sprayed an antibody to the BST beta-lactam receptor. A raw-milk sample, between 0.1-1.0 ml preferably, is applied to the sample pad by pipette, and the test strip is incubated at 55°C. After about eight minutes, test strip 10 is removed from the incubator and analyzed. If test zone 38 is darker or the same color as control zone 40 line, the sample is negative, and, if test zone 38 is lighter than control zone 40, the sample is positive.

Test results are shown in Table 1 as follows:

**Table 1. Beta-lactam assay in milk using lateral flow test device.**

| Number of Assays | Sample | Result |
|---|---|---|
| 30 | zero control | all negative |
| 10 | penicillin G at 5 ppb | all positive |
| 10 | penicillin G at 4 ppb | 5 positive, 5 negative |
| 10 | penicillin G at 3 ppb | 3 positive, 7 negative |
| 10 | ampicillin at 6 ppb | all positive |
| 10 | ampicillin at 4 ppb | all positive |
| 10 | ampicillin at 3 ppb | 5 positive, 5 negative |
| 10 | amoxicillin at 6 ppb | all positive |
| 10 | amoxicillin at 4 ppb | 8 positive, 2 negative |
| 10 | amoxicillin at 3 ppb | 4 positive, 6 negative |
| 10 | ceftiofur at 30 ppb | 3 positive, 7 negative |
| 10 | ceftiofur at 40 ppb | 8 positive, 2 negative |
| 10 | ceftiofur at 50 ppb | 10 positive |
| 10 | cephapirin at 12 ppb | 2 positive, 8 negative |
| 10 | cephapirin at 15 ppb | 5 positive, 5 negative |
| 10 | cephapirin at 20 ppb | 10 positive, 0 negative |

The described test is an inhibition-type assay. Analyte in the sample binds with a beta-lactam binding protein/mobile-phase composition probe and inhibits binding to a stationary beta-lactam bound to the surface of the membrane. Addition of a specific monoclonal antibody to ceftiofur has altered its inhibition level from approximately five ppb to between 40 and 50 ppb. Addition of a specific monoclonal antibody to cephapirin has reduced its sensitivity from approximately 3-5 ppb to between 15 to 20 ppb.

The test device can be used with test strips for detecting a variety ofanalytes, such as toxins like alfatoxins, pesticides such as organophosphates and carbamates; as well as beta-lactams, such as penicillin, ampicillin, amoxicillin, cloxacillin, dicloxacillin, oxacillin, ceftiofur, and cephapirin; tetracyclines, such as chlortetracycline, oxytetracycline and tetracycline; sulfonamides, such as sulfamethazine, sulfadimethoxine, sulfamerazine, sulfathiazole and sulfadiazine; macrolides, such as erythromycin, spiramycin and tylosin; aminoglycosides, such as gentamicin, neomycin and DH/striptomycin; and others such as dapsone, chloramphenicol, novobiocin, spectinomicin and trimethoprim, to detect the maximum residue-analyte limits in the sample. Most of the elements for each test are the same except the chemistries of the mobile phase, test zone and control zone, which are tailored to the specific analyte detection.

As the sample flows from stationary-phase membrane 36 into disposal zone 43 (until absorbent pad saturation), the unreacted tagged-receptor is captured in the reaction zone by an immobilized group representative analyte. Chemical residue in the sample reacts with the tagged-receptor making it unreactive to the test line. Thus, the more residue in the sample, less signal is detected in the test zone.

Stationary-phase membrane is constructed from highly porous matrix suitable for viscous samples, such as milk or meat extracts. In each zone, a combination of soluble polymers is embedded (e.g. proteins, polyethylene glycol, etc.) to control the kinetics of mobility of the sample from the mobile-phase composition to the reaction zone and in the reaction zone itself.

Data were generated with microbial beta-lactam receptor, specific antibodies for sulfamethazine, tretracycline and aflatoxin (pt CHII AOAC) to detect for the presence of corresponding residues in milk or other matrices such as serum. Levels of 3-5 ppb penicillin G (PEN G) and 5-20 ppb cephapirin, 30-100 ppb oxytetracycline (OXT), 10-100 ppb sulfamethazine (SMZ) and 2-40 ppb aflatoxin B1 were detected with these experiments.

An incubator with adjustable temperature ranging up to 70°C generally is preferred. The test device can employ a portable colorimeter, or refractive fluorometer, or infrared reader. In a preferred embodiment, the test device includes a reader that is used to read a test strip that contains two lines. The control line is a reference line that insures that the test has been run correctly. The control line is also used as a reference when the reader determines if the sample is positive or negative. The test line indicates the concentration of the substance being tested. The darker the test line the higher the concentration of the substance in the sample. The reader includes two components, a controller and a meter.

The meter reads the strip when the strip is inserted into the meter and the meter is given the command to read the strip. The meter then strobes a series of light emitting diodes (LED), preferably, seven. The light emitted from the LED's is bounced off the strip being read. The light is then reflected onto a 128x1 Opto Sensor. The sensor sends 128 date values representing the intensity of the light at each of the 128 pixels to an on board microcontroller. The pixel date is stored in the meter's memory. The dark areas of the strip have a lower value than do the light areas. This information is later used to calculate the intensity of the two lines being read.

The controller sends a command to the meter to request the date read by the meter. The controller performs calculations on the date to determine the intensity of the two lines. If the test line is darker than the control line then the test is said to have a negative result. If the test line is lighter than the control line then the test is said to have a positive result. The controller displays to the user the result as well as a raw value representing the difference in the intensity of the two lines.

Integration of incubator with fiber optics to read the results can provide the test with full automation.

The test unit, such as in blister pack form, is placed in an incubator which is heated to about 56.5°C ± 1°C. The tape is peeled back and a liquid sample, 0.3 ml, is added to the sample well and the tape is resealed. The test unit is incubated for at least five minutes.

Once the sample is added to the sample well, it is absorbed by the sample sponge which expands inside the well. The top portion of the plastic well prevents the sponge from expanding fully. The pressure of the sponge against the well on the top and the mobile-phase support on the bottom gives some added force to propel the liquid sample up the test strip at a faster rate than would otherwise occur. The sponge expands and the sample next moves onto the mobile-phase support and interacts with the mobile-phase composition. The mobile-phase composition starts to move onto the nitrocellulose. During this time incurred residues or analytes in the sample bind to the receptor or antibody attached to the mobile-phase conjugate.

When the mobile-phase composition reaches the test zone, the free labeled receptor binds to the test zone resulting in a dark bottom line. Receptor or antibody with bound analyte does not bind to the test line resulting in a noncolored or light colored test zone line. This is a sequential inhibition-type assay where the compound of concern does not bind to the test zone. The mobile-phase composition moves past the control zone and onto an absorbent pad which serves as a reservoir to catch unbound mobile-phase composition.

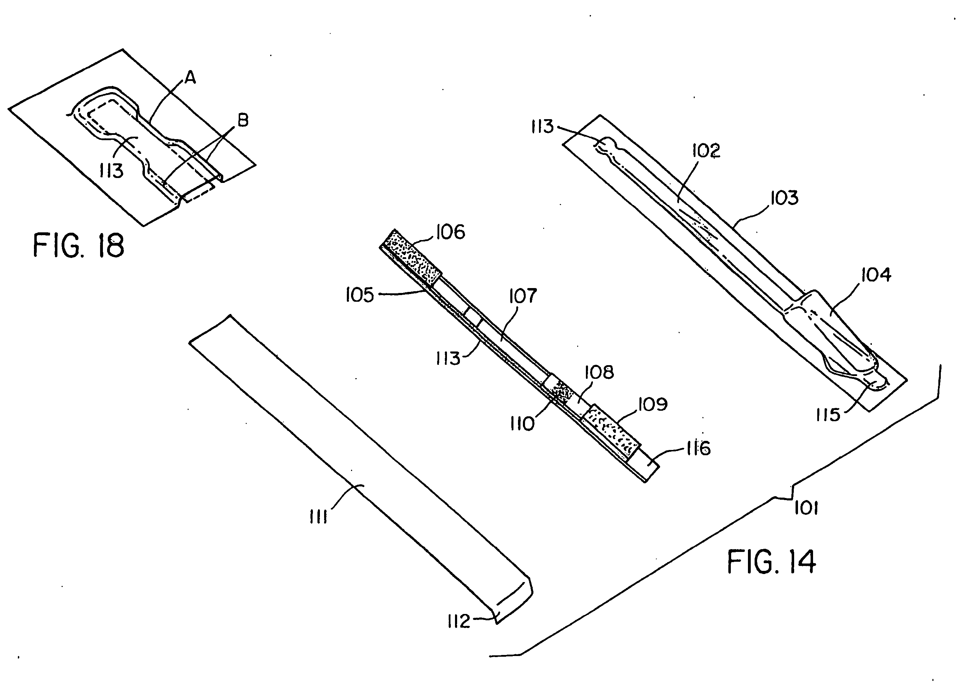

Figures 14-17 illustrate an embodiment of the device 101 in transparent blister package 103 which includes transparent-tape plastic seal strip 111, to enclose test strip 105 therein. Blister package 103 includes an elongated cavity to hold strip 105 and expansion cavity-housing 104 to form a generally tooth-brush shaped cavity within plastic blister package 103 and strip 105. As shown, expansion cavity-housing 104 is triangularly shaped. The blister package 103 includes one movement restriction zone 114 surrounding the disposal pad 106 and another movement restriction zone 115 surrounding adhesive backing 113 at the point at which backing 113 protrudes before sample absorbing sponge 109. Movement restriction zones 114, 115 form pinch points which secure strip 105 within blister package 103. The device, therefore, is designed so that one location at which narrowing occurs is at disposal pad 106 in which zone there is located an absorbent material which acts as an absorbent pad. Preferably, the device is designed, and placement of components located so that, approximately one cm of adhesive backing protrudes before the sample sponge pad contained within the sample application zone. The strip is, therefore, secured in place at either one or both ends, thereby allowing unimpeded sample flow.

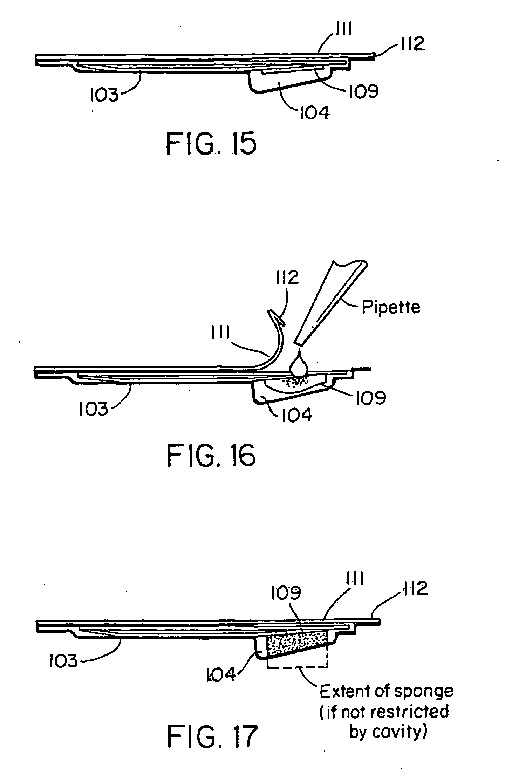

Figure 15 shows a side sectional view of blister-package test device 101 prior to use. Figure 15 shows blister-package test device 101 with one end peeled back by peel tab 112, to expose expansion housing cavity 104 and dry filter-absorbent sponge pad 109 of test strip 105, so that a defined amount of a liquid sample can be added, for example, by pipette, as shown. Figure 17 illustrates test device 101 after addition of the liquid sample, and with peel tab 112 resealed and with the sponge pad 109 fully expanded by the liquid sample within housing cavity 104 and ready to incubate.

Figure 18 is an enlarged view of absorbent pad 109 of the test device. Figure 18 illustrates the narrowing of the inner walls of the housing in zone A to form movement restriction zone 114 securing the adhesive backing 113 and thereby strip 105 (not shown in Figure 18) is held in place within the plastic blister 113. Figure 18 also illustrates the air space zone B existing between strip 105 and adhesive backing 113 which allows consistent flow of sample along the strip.

## Claims

1. A method employing a lateral flow test strip for determining whether one or more members of an analyte family comprising beta-lactam antibiotics is present in a test sample, which method comprises:
a) allowing said sample to contact a microbial beta-lactam receptor, wherein said receptor is **characterized by** an ability to bind to members of said analyte family, so as to allow said receptor to bind one or more members of said analyte family in said sample;
b) allowing said sample to contact an additional analyte binder, said additional analyte binder having binding specificity for at least one member of said analyte family to which said receptor binds, so that there is competition between the receptor and the additional analyte binder for binding to said at least one analyte, said competition causing a decrease in test sensitivity, relative to said at least one member, to a selected threshold level; and
c) detecting said one or more members of said analyte family.

2. A method according to Claim 1 further comprising detecting said one or more members by comparative colour observation of a test zone and control zone on the lateral-flow test strip.

3. A method according to Claim 1 or Claim 2 wherein the receptor comprises a labelled receptor.

4. A method according to any preceding claim further comprising allowing said sample to contact a test zone on the lateral-flow test strip, said test zone comprising a test zone binder wherein said test zone binder comprises an analyte conjugate which binds to the receptor if said receptor is not bound to an analyte from the sample.

5. A method according to Claim 4 further **characterized in that** the method is for determining the level of beta-lactam in milk.

6. A method according to any preceding claim further comprising allowing said sample to contact a control zone on the lateral flow test strip, said control zone comprising a control-zone binder that binds to said receptor.

7. A method according to any preceding claim wherein said method comprises an assay for beta-lactams in milk at a safe level and wherein said receptor is a bacterial protein.

8. A method according to any preceding claim wherein said additional analyte binder is an antibody.

9. A method according to claim 8 wherein said additional binder is a monoclonal antibody specific to a member of the analyte family.

## Patentansprüche

1. Verfahren, bei dem ein Lateralströmung-Teststreifen eingesetzt wird, um zu bestimmen, ob ein oder mehrere Mitglied(er) einer Analytfamilie, die beta-Lactam-Antibiotika umfasst, in einer Testprobe vorliegt bzw. vorliegen, wobei das Verfahren umfasst:
a) Erlauben der Probe, einen mikrobiellen beta-Lactam-Rezeptor zu kontaktieren, wobei der Rezeptor durch ein Vermögen **gekennzeichnet** ist, an Mitglieder der Analytfamilie zu binden, so dass der Rezeptor ein oder mehrere Mitglied(er) der Analytfamilie in der Probe binden kann,
b) Erlauben der Probe, ein zusätzliches Analytbindungsmittel zu kontaktieren, wobei das zusätzliche Analytbindungsmittel eine Bindungsspezifität für mindestens ein Mitglied der Analytfamilie aufweist, an das der Rezeptor bindet, so dass eine Konkurrenz zwischen dem Rezeptor und dem zusätzlichen Analytbindungsmittel um das Binden an den mindestens einen Analyten vorliegt, wobei die Konkurrenz eine Verminderung der Testempfindlichkeit, bezogen auf das mindestens eine Mitglied, auf einen ausgewählten Schwellenwert verursacht, und
c) Nachweisen des einen Mitglieds oder der mehreren Mitglieder der Analytfamilie.

2. Verfahren nach Anspruch 1, das ferner das Nachweisen des einen Mitglieds oder der mehreren Mitglieder durch eine vergleichende Farbuntersuchung einer Testzone und einer Kontrollzone auf dem Lateralströmung-Teststreifen umfasst.

3. Verfahren nach Anspruch 1 oder Anspruch 2, bei dem der Rezeptor einen markierten Rezeptor umfasst.

4. Verfahren nach einem der vorhergehenden Ansprüche, das ferner das Erlauben der Probe, eine Testzone auf dem Lateralströmung-Teststreifen zu kontaktieren, umfasst, wobei die Testzone ein Testzonenbindungsmittel umfasst, wobei das Testzonenbindungsmittel ein Analytkonjugat umfasst, das an den Rezeptor bindet, wenn der Rezeptor nicht an einen Analyten von der Probe gebunden ist.

5. Verfahren nach Anspruch 4, das ferner **dadurch gekennzeichnet ist, dass** das Verfahren zur Bestimmung der Konzentration von beta-Lactam in Milch dient.

6. Verfahren nach einem der vorhergehenden Ansprüche, das ferner das Erlauben der Probe, eine Kontrollzone auf dem Lateralströmung-Teststreifen zu kontaktieren, umfasst, wobei die Kontrollzone ein Kontrollzonenbindungsmittel umfasst, das an den Rezeptor bindet.

7. Verfahren nach einem der vorhergehenden Ansprüche, wobei das Verfahren einen Test bezüglich beta-Lactamen in Milch auf einem sicheren Niveau umfasst und bei dem der Rezeptor ein bakterielles Protein ist.

8. Verfahren nach einem der vorhergehenden Ansprüche, bei dem das zusätzliche Analytbindungsmittel ein Antikörper ist.

9. Verfahren nach Anspruch 8, bei dem das zusätzliche Bindungsmittel ein monoklonaler Antikörper ist, der für ein Mitglied der Analytfamilie spezifisch ist.

## Revendications

1. Procédé employant une bandelette réactive à flux latéral pour déterminer si, oui ou non, un ou plusieurs membres d'une famille d'analytes composée d'antibiotiques à noyau beta-lactame est/sont présents dans un échantillon à tester, lequel procédé comprend des étapes consistant à :
a) permettre audit échantillon de venir au contact d'un récepteur microbien de beta-lactames, ledit récepteur étant **caractérisé par** une aptitude à se lier à des membres de ladite famille d'analytes, afin de permettre la liaison dudit récepteur avec un ou plusieurs membres de ladite famille d'analytes dans ledit échantillon ;
b) permettre audit échantillon de venir au contact d'un liant supplémentaire pour analytes, ledit liant supplémentaire pour analytes ayant une spécificité de liaison pour au moins un membre de ladite famille d'analytes auquel se lie ledit récepteur, de façon qu'il y ait concurrence entre le récepteur et le liant supplémentaire pour analytes, ladite concurrence provoquant une baisse de sensibilité du test, en ce qui concerne ledit au moins un membre, jusqu'à un niveau de seuil choisi ; et
c) détecter ledit/lesdits membre(s) de ladite famille d'analytes.

2. Procédé selon la revendication 1, comprenant en outre une étape consistant à détecter ledit/lesdits membre(s) par observation comparative de couleurs d'une zone de test et d'une zone témoin sur la bandelette réactive à flux latéral.

3. Procédé selon la revendication 1 ou 2, dans lequel le récepteur est constitué par un récepteur marqué.

4. Procédé selon l'une quelconque des revendications précédentes, comprenant en outre une étape consistant à permettre audit échantillon de venir au contact d'une zone de test sur la bandelette réactive à flux latéral, ladite zone de test comportant un liant de zone de test, ledit liant de zone de test comportant un conjugué d'analyte qui se lie au récepteur si le récepteur n'est pas lié à un analyte de l'échantillon.

5. Procédé selon la revendication 4, **caractérisé en outre en ce que** le procédé sert à déterminer le taux de beta-lactames dans le lait.

6. Procédé selon l'une quelconque des revendications précédentes, comprenant en outre une étape consistant à permettre audit échantillon de venir au contact d'une zone témoin sur la bandelette réactive à flux latéral, ladite zone témoin comportant un liant de zone témoin qui se lie audit récepteur.

7. Procédé selon l'une quelconque des revendications précédentes, ledit procédé comportant un dosage de béta-lactames dans le lait à un niveau sans danger, ledit récepteur étant une protéine bactérienne.

8. Procédé selon l'une quelconque des revendications précédentes, dans lequel ledit liant,supplémentaire pour analytes est un anticorps.

9. Procédé selon la revendication 8, dans lequel ledit liant supplémentaire est un anticorps monoclonal spécifique d'un membre de la famille d'analytes.
